# EUROPEAN PATENT SPECIFICATION

(11) **EP 3 496 725 B1**
(45) Date of publication and mention of the grant of the patent: **05.10.2022**
(21) Application number: 17840377.0
(22) Date of filing: 11.08.2017
(51) Int. Cl.: A61K 31/55, A61K 31/4535, A61K 31/075, A61K 31/40, A61K 31/138, A61K 31/085, A61K 31/277, A61K 31/565, A61K 31/566, A61K 45/06, A61P 25/00, A61P 25/28

(54) **REMYELINATION THERAPIES**
THERAPIEN ZUR REMYELINISIERUNG
TRAITEMENTS DE REMYÉLINISATION

(30) Priority: 12.08.2016 US 201662374270 P; 06.12.2016 US 201662430357 P
(43) Date of publication of application: 19.06.2019
(73) Proprietor: The Regents of the University of California, Oakland, CA 94607 (US)
(72) Inventor: BOVE, Riley, San Francisco, California 94158 (US); CHAN, Jonah, San Francisco, California 94158 (US); GREEN, Ari, San Francisco, California 94158 (US); SHEN, Yun-An, San Francisco, CA 94132 (US); RANKIN, Kelsey, San Francisco, California 94158 (US)
(74) Representative: Appleyard Lees IP LLP
(86) International application number: PCT/US2017/046624
(87) International publication number: WO 2018/031945

(56) References cited:
- WO-A1-96/05823
- WO-A1-2015/181676
- WO-A2-2004/062653
- WO-A2-2010/008588
- WO-A2-2018/022904
- GONZALEZ G ET AL: "TAMOXIFEN PROMOTES CNS REMYELINATION BY MODULATION OF THE PKC SIGNALLING PATHWAY IN OLIGODENDROCYTE PRECURSOR CELLS (OPCS)", GLIA, vol. 61, no. Suppl. 1, July 2013 (2013-07) , pages S200-S201, XP002798047, & 11TH EUROPEAN MEETING ON GLIAL CELL FUNCTION IN HEALTH AND DISEASE; BERLIN, GERMANY; JULY 03 -06, 2013 ISSN: 0894-1491
- María-Angeles Arevalo et al.: "Actions of estrogens on glial cells: Implications for neuroprotection", Biochimica et Biophysica Acta, vol. 1800 7 October 2009 (2009-10-07), pages 1106-1112, XP002798048, Retrieved from the Internet: URL:https://www.sciencedirect.com/science/ article/pii/S0304416509002888?via%3Dihub [retrieved on 2020-03-03]
- KUMAR SHALINI ET AL: "Estrogen receptor [beta] ligand therapy activates PI3K/Akt/mTOR signaling in oligodendrocytes and promotes remyelination in a mouse model of multiple scler", NEUROBIOLOGY OF DISEASE, ELSEVIER, AMSTERDAM, NL, vol. 56, 17 April 2013 (2013-04-17), pages 131-144, XP028560220, ISSN: 0969-9961, DOI: 10.1016/J.NBD.2013.04.005
- D. K. CRAWFORD ET AL: "Oestrogen receptor beta ligand: a novel treatment to enhance endogenous functional remyelination", BRAIN, vol. 133, no. 10, 1 October 2010 (2010-10-01), pages 2999-3016, XP055131983, ISSN: 0006-8950, DOI: 10.1093/brain/awq237
- MRINMAY CHAKRABARTI ET AL: "Estrogen receptor agonists for attenuation of neuroinflammation and neurodegeneration", BRAIN RESEARCH BULLETIN., vol. 109, 1 October 2014 (2014-10-01), pages 22-31, XP55672797, GB ISSN: 0361-9230, DOI: 10.1016/j.brainresbull.2014.09.004
- RANKIN KELSEY A ET AL: "Selective Estrogen Receptor Modulators Enhance CNS Remyelination Independent of Estrogen Receptors.", THE JOURNAL OF NEUROSCIENCE : THE OFFICIAL JOURNAL OF THE SOCIETY FOR NEUROSCIENCE 20 MAR 2019, vol. 39, no. 12, 20 March 2019 (2019-03-20), pages 2184-2194, XP002798049, ISSN: 1529-2401
- BEBO JR., B. F. ET AL.: 'Treatment with selective estrogen receptor modulators regulates myelin specific T-cells and suppresses experimental autoimmune encephalomyelitis' GLIA, [Online] vol. 57, 20 November 2009, pages 777 - 790, XP009128047 Retrieved from the Internet: <URL:https://doi.org/10.1002/glia.20805>
- ACKERMAN, S. D. ET AL.: 'The adhesion GPCR Gpr56 regulates oligodendrocyte development via interactions with Ga 12/13 and RhoA' NATURE COMMUNICATIONS, [Online] vol. 6, no. 6122, 21 January 2015, pages 1 - 14, XP055425931 Retrieved from the Internet: <URL:doi:10.1038/ncomms7122>
- AREVALO, M. A. ET AL.: 'Selective oestrogen receptor modulators decrease the inflammatory response of glial cells' JOURNAL OF NEUROENDOCRINOLOGY, [Online] vol. 24, 12 May 2011, pages 183 - 190, XP055463589 Retrieved from the Internet: <URL:https://doi.org/10.1111/j.1365-2826.20 11.02156.x>

## Description

CROSS-REFERENCE TO RELATED APPLICATIONS: This application claims the benefit of priority to United States Provisional Application Serial Number 62/374,270 entitled "Remyelination Therapy," filed August 12, 2016, and United States Provisional Application Serial Number 62/430,357 entitled "Remyelination Therapy," filed December 6, 2016.

### Background of the Invention

In various demyelinating diseases, such as Multiple sclerosis (MS) the myelin coating that surrounds nerve fibers is attacked and damaged by the immune system or other factors. Myelin repair, or remyelination, is carried out by myelin-producing oligodendrocytes. In healthy animals, following demyelination processes, oligodendrocyte progenitor cells (OPCs) are activated and mature into myelin-producing oligodendrocytes, which wrap the axons and reapply myelin to damaged areas.

However, in various demyelinating diseases such as MS, the remyelination process is deficient and myelin damage accumulates over time, leading to severe degeneration. It is generally accepted that remyelination failure is not a result of impaired OPC recruitment and/or migration to demyelinated tissues. Instead, it is believed that a failure of OPC differentiation and membrane wrapping is the critical barrier impeding myelin repair in demyelinating conditions.

Current MS treatments address the underlying inflammatory component of the disease, but to date, there are no approved therapies for axon repair or remyelination. Accordingly, there is an ongoing and significant need in the art for effective remyelination therapies.

Gonzalez G. et al.; Glia; 2013 (Suppl.1); pages S200-S201 discloses that tamoxifen promotes CNS remyelination by modulation of the PKC signalling pathway in oligodendrocyte precursor cells (OPCs).

WO 2010/008588 discloses that tamoxifen is able to promote OPC differentiation and CNS remyelination in demyelination condition, such as MS.

### Summary of the Invention

The inventors of the present disclosure have advantageously developed novel therapies that promote remyelination. In a first aspect, the inventors of the present disclosure have identified certain selective estrogen receptor modulators (SERMs) that promote remyelination. In another aspect, the inventors of the present disclosure have determined that GPR56, a G-coupled protein receptor active in certain cells, is a mediator of the remyelination process and that agonists of this receptor can promote remyelination and associated processes in demyelinated tissues.

Additionally, the inventors of the present disclosure have determined that administration of remyelinating SERMs or GPR56 agonists in combination with estrogenic substances further promotes remyelination.

In one aspect, the scope of the invention encompasses novel therapeutic compositions comprising remyelinating SERMs. In one aspect, the scope of the invention encompasses novel therapeutic compositions comprising remyelinating SERMs which also act as GPR56 agonists

In another aspect, the scope of the invention encompasses remyelinating SERMs for the treatment of demyelinating conditions, promoting remyelination, and promoting the differentiation of oligodendrocyte precursors.

According to the present invention, the remyelinating SERM is bazedoxifene or clomifene.

### Brief Description of the Drawings

Fig. 1. BIMA micropillar assay results showing the percentage of rings stained positive for PDGFRα (indicative of OPCs) or myelin basic protein (MBP) (indicative of myelinating oligodendrocytes) in response to treatment with various agents.
Fig. 2. BIMA micropillar assay results showing the percentage of rings stained positive for MBP for BZA-treated cells at different dosages, with and without estradiol.
Fig. 3. Adenomatous polyposis coli (APC-a maker of mature oligodendrocytes) positive cell density and Olig2 (a marker of oligodendroglial lineage) cell density in rat corpus callosum following lysolecithin-induced demyelination and subsequent treatment with BZA.
Fig. 4A, 4B, 4C, and 4D. Fig 4A, 4B, 4C, and 4D depict the percentage of MBP-positive cells following BZA treatment of isolated OPCs grown in culture. Fig 4A depicts the BZA response in wild-type cells, Fig 4B depicts the BZA response in ERa knockout cells, Fig. 4C depicts the BZA response in ERβ knockout cells, and Fig. 4D depicts the BZA response in ERa /ERβ double knockout cells.

### Detailed Description of the Invention.

**Demyelinating Diseases.** In one aspect, the various embodiments of the invention are directed to the treatment of a demyelinating condition. A demyelinating condition, as used herein, refers to any disease state, autoimmune disorder, or process wherein demyelination, i.e. damage to the protective myelin sheath that surrounds nerve fibers, is occurring. Typically, such damage is caused by autoimmune processes of unknown origin.

In one embodiment, the demyelinating condition is a condition of the central nervous system. In one embodiment, the demyelinating disease is a myelinoclastic disorder. In one embodiment, the demyelinating condition is MS. In other embodiments, the demyelinating disease may be Devic's disease, an inflammatory demyelinating disease, or an acute disseminated encephalomyelitis. The demyelinating condition may comprise a leukodystrophic disorder. The demyelinating condition may comprise a central nervous system neuropathy, central pontine myelinolysis, or progressive multifocal leukoencephalopathy. The compositions and methods of the invention may also be applied to promote remyelination in demyelinating conditions of the peripheral nervous system.

**Remyelinating Compositions.** The inventors of the present disclosure have identified certain compositions of matter that can act as remyelinating agents. The scope of the invention encompasses the administration of a what will be referred to herein as a "remyelinating composition." A remyelinating composition comprises one or more remyelinating agents.

In a first aspect, the remyelinating composition comprises a SERM with remyelinating activity. Such compositions will be referred to herein as "remyelinating SERMs." SERMs are compositions known to act on estrogen receptors, with tissuespecific effects, for example, acting as estrogenic agents in some tissues while being anti-estrogenic in other tissues.

According to the present invention, the remyelinating SERM is bazedoxifene or clomifene.

In a second non-claimed aspect, the remyelinating composition comprises a GPR56 agonist. GPR56, as known in the art, is G-coupled protein receptor active in various tissues. The GPR56 protein sequence has accession number Q9Y653, and a GeneCard ID of GC16P057610 and an NCBI Reference Sequence of NG_011643.1. GPR56 is also known as Adhesion G Protein-Coupled Receptor G1, and as TM7XN1.

GPR56 has previously been implicated in brain cortical patterning during development, ovarian development, cell adhesion, cell-cell interactions, and the maintenance of hematopoietic stem cells and/or leukemia stem cells in bone marrow niche. No role for GPR56 in myelination has previously been reported.

The inventors of the present disclosure have advantageously determined that GPR56 is implicated in remyelination. Specifically, certain remyelinating compositions have been identified which comprise SERMs and which also act as GPR56 agonists. This discovery provides the art with various methods of treatment, secondary uses of known compounds, and other valuable inventions which may be applied to promote remyelination.

Various non-claimed aspects of the present disclosure are directed to activation of GPR56 by a GPR56 agonist. An agonist, as used herein, refers to any composition of matter which binds to GPR56 and induces one or more GPR56-associated myelination processes in oligdendroglia or other cells. The GPR56 Agonist may be a small molecule, growth factor, polypeptide, nucleic acid, or any other chemical or biological composition of matter.

GPR56 agonists, as used herein, will further refer to agonists, activators, and enhancers of GPR56's downstream effectors, i.e., activators of species which are regulated by GPR56 activation and which contribute to the remyelination response induced by GPR56 activation.

The inventors of the present disclosure have advantageously identified several remyelinating compositions. The remyelinating composition comprise remyelinating SERMs and also comprise putative GPR56 agonists.

A first remyelinating composition of the invention is bazedoxifene. Bazedoxifene (BZA) is a selective estrogen receptor modulator (SERM) which, in combination with conjugated estrogens, has been approved for the treatment of postmenopausal osteoporosis and of menopausal hot flashes. BZA in combination with conjugated estrogens is well tolerated, for example, having lesser side effects than tamoxifen.

A second non-claimed remyelinating composition is raloxifene. Raloxifene is an FDA-approved drug, used in the prevention of osteoporosis and as a breast cancer preventative in postmenopausal women.

A third remyelinating composition (according to the invention) is clomifene. Clomifene is an FDA-approved drug, used in fertility treatment in women.

A fourth non-claimed remyelinating composition is toremifene. Toremifene is an-FDA approved drug, used in the treatment of breast cancer.

A fifth non-claimed remyelinating composition is lasofoxifene. Lasofoxifene an EU-approved drug, and has shown efficacy in treating osteoporosis, breast cancer prevention, and vaginal atrophy.

A sixth non-claimed remyelinating composition is ospemifene. Ospemifene is FDA-approved for the treatment of dyspareunia.

A seventh non-claimed remyelinating composition is diarylpropionitrile. Diarylpropionitrile is an estrogen receptor agonist and is an activator of the endogenous oxytocin system.

An eighth non-claimed remyelinating composition agonist is tamoxifen. Tamoxifen is widely used in the treatment of certain breast cancers.

Interestingly, each of the afore-listed compounds is a SERM. However, the inventors of the present disclosure have determined that the remyelination effects of these SERMs is mediated independently of either the Alpha or Beta estrogen receptors. As described in the Examples, below, remyelination is induced by these compounds in oligodendroglia even in cells wherein both forms of the ER have been knocked out. Accordingly, the remyelination effects induced by these compounds are largely independent of ER activity and are believed to be largely or wholly by action of GPR56 activation.

The scope of the invention encompasses various remyelinating compositions compositions. In one embodiment, the scope of the invention encompasses a pharmaceutical composition comprising remyelinating composition for the treatment of a demyelinating condition. In one embodiment, the remyelinating composition is a remyelinating SERM. According to the present invention, the remyelinating composition is selected from the group consisting of bazedoxifene, clomifene, which are remyelinating SERMs and act as GPR56 agonists.

**Co-Administration with Estrogens**. The inventors of the present disclosure have determined that the remyelinating effects of the remyelinating compositions are enhanced when co-administered with estrogenic substances. It is known in the art that MS has an estrogenic component, based on various associations such as an increased incidence of MS in women vs. males and decreased incidence of MS onset in postmenopausal women. However, the role of estrogenic hormones in MS is not clear, and administration of estrogenic hormones alone has not been shown to effectively treat demyelinating conditions. Accordingly, the surprising enhancement of remyelination by estrogenic hormones in combination with remyelinating compositions provides the art with a remyelination therapy of increased potency.

The co-administered estrogenic agent may comprise any estrogen hormone or estrogenic substance, for example, such as estrogen, estradiol, conjugated estrogens, estriol, estrogen mimics, estrone, ethynil estrogen, and estrogen agonists.

**Methods of the Invention**. The scope of the invention encompasses various uses of utilizing remyelinating compositions to promote remyelination processes. The uses disclosed herein encompass the administration of a remyelinating composition to a subject.

Such administration may encompass the administration of a pharmaceutically effective amount of the remyelinating composition, i.e. an amount sufficient to have a measurable effect on one or more remyelinating processes. For example, remyelinating composition dosages which result in physiological concentrations of 1 nM to 1 mM may be used, for example in the range of 5 nM to 500 nM. For estrogenic agents combined with remyelinating compositions, exemplary dosages include those which result in a physiological concentration in the range of 1 to 100 micromolar.

The subject of the administration may be any animal, for example, a human, a veterinary subject, or a test animal. In one embodiment, the subject is a human that is afflicted with a demyelinating condition or is at risk of having a demyelinating condition and is in need of treatment therefor. In a non-claimed aspect, the scope of the disclosure also extends to the administration of remyelinating compositions to cells, cell cultures, and explanted tissues. In one aspect, the scope of the disclosure encompasses the administration of a remyelinating composition to oligodendroglia in a micropillar assay, as known in the art.

In some embodiments, the administration of a remyelinating composition will be referred to as increasing, enhancing, or otherwise changing the magnitude of a myelination-associated process. Such change may be defined with respect to the magnitude of the myelination-associated process observed in untreated controls or in subjects prior to treatment.

In one aspect, the scope of the invention encompasses a remyelinating composition for use in the treatment of a demyelinating condition in a subject, wherein the remyelinating composition is a remyelinating SERM, as defined in claim 1. Treatment, as used herein, may include, for example; curing a demyelinating condition; ameliorating symptoms associated with demyelination (e.g. nerve signal disruption, axonal damage, and neurodegeneration); slowing the progression of a demyelinating condition; preventing or delaying the onset of a demyelinating condition in an at-risk subject; preventing further loss of myelin; or restoring myelin lost prior to treatment. Such treatment or use may be in combination with the administration one or more estrogenic agents.

In one aspect, the scope of the invention encompasses a a remyelinating SERM such as defined in claim 1 for use in a method of enhancing remyelination of axons in a subject suffering from a demyelinating condition. Enhancement, as used herein, refers to increasing the degree of remyelination, for example, increasing the thickness of the myelin sheath on axons, increasing the rate or prevalence of axon wrapping, increasing the rate of remyelination, or increasing the number of remyelinated axons in a treated area. Such administration may be in combination with one or more estrogenic agents.

In one aspect, the scope of the invention encompasses a a remyelinating SERM such as defined in claim 1 for use in a method of enhancing the differentiation of OPCs into myelinating oligodendrocytes in a subject. Enhanced differentiation, as used herein, may refer to in increase in any measure of OPC differentiation into myelin-producing oligodendrocyte cells, including, for example, increasing the proportion of differentiated oligodendrocytes to OPC's, for example, an increase in MBP expressing cells or a decrease in the proportion of PDGFRα expressing cells. Such administration may be in combination with one or more estrogenic agents.

In another aspect, the scope of the invention encompasses a method of using a remyelinating composition of claim 1 for the manufacture of a medicament for use in the treatment of a demyelinating condition. For example, a pharmaceutical composition for the treatment of a demyelinating condition may comprise one or more remyelinating compositions formulated or mixed in combination with one or more additional pharmaceutically acceptable elements. For example, remyelinating compositions may be combined with compositions such as carriers, excipients, diluents, buffers, salts, or release-modulating agents, as well as estrogenic compositions.

### Examples.

**Example 1. Identification of Remyelinating SERMs.** Various SERMs and other compounds were tested for their remyelination effects using the BIMA (Binary Indicant for myelination using Micropillar Arrays) assay, a functional high-throughput screen utilizing freestanding micropillar arrays of compressed silica around which myelin "rings" of membrane wrapping by oligodendroglia can be visualized in crosssection. BIMA allows testing of compounds' direct influences on oligodendroglia without indirect effects from neurons and other factors.

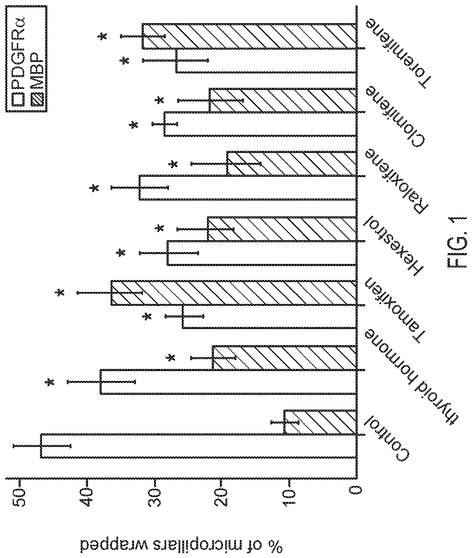

Micropillars were cultured with OPCs and the number of MBP-positive or PDGFRα-positive rings in each field of 100 micropillars was determined. Results are depicted in Fig. 1. Error bars represent mean ± s.e.m. *P < 0.05, significance based on Student's t-test with the respective controls. Seven additional SERMs or estrogen derivatives were screened at concentrations between 500nM-1µM without any significant effects on oligodendrocyte differentiation or membrane wrapping.

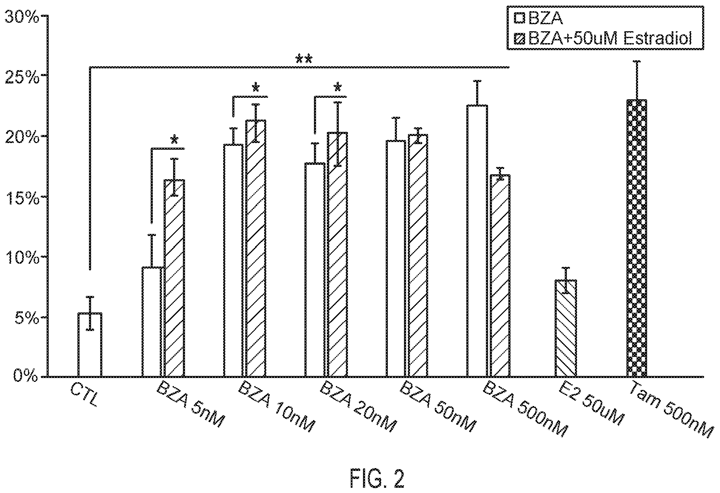

**Example 2. BZA promotes oligodendrocyte differentiation** Purified OPCs were cultured and treated with different concentrations of BZA alone or with estradiol for 48 hours and immunostained for MBP, PDGFRα and DAPI. Quantification of the percentage of MBP-positive cells after treatment is depicted in Fig. 2. Error bars represent mean ± s.e.m., *P < 0.05, **P < 0.01, significance based on Student's t-test with the respective controls.

**Example 3. OPC Differentiation.** To assess the effect of selected SERMs on myelination, OPCs were derived from WT P7 rat pups using previously validated methodology, cultured in isolation, and treated with a select group of SERMs at a preliminary concentration of 500nM for 48 hours. Cells were then permeabilized and immunostained for MBP (oligodendrocytes), PDGFRα (OPCs), and DAPI (cell bodies). Each SERM tested ((2,3-bis(4-hydroxyphenyl)-propionitrile, BZA and tamoxifen) significantly enhanced OPC differentiation (*p<0.05; **p<0.01; ***p<0.001) at this concentration. Additionally, OPCs were co- cultured with dorsal root ganglion neurons (DRGs) and subsequently treated with 500nM BZA every 3 days. Co-cultures were fixed, permeabilized and stained. BZA significantly (p<0.001) enhanced OPC differentiation and subsequent myelination in the co-culture system, showing strong effects on OPC differentiation and myelination.

**Example 4. BZA treatment on human ESC-derived OPCs.** Human OPCs were generated from human ESCs and cultured for 10 days in the presence or absence of BZA (500 nM). The cells were then stained for O4 and MBP. There was a significant increase in the number of MBP-positive oligodendrocytes upon treatment with BZA.

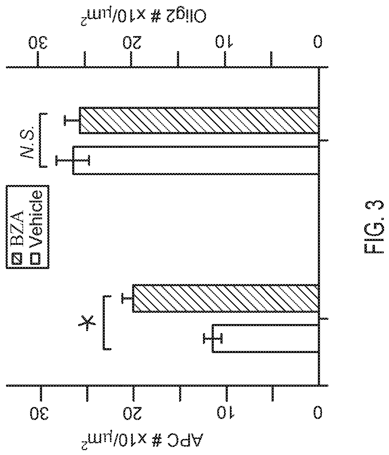

**Example 5. Effects of BZA on OPC Differentiation.** Lysolecithin was injected in the corpus callosum of mice and analyzed at 6 days after injection, showing a demyelinated lesion area. Mice were treated with either BZA (10mg/kg) or vehicle control for 7 days after injection of lysolecithin. Mice were euthanized at 10 days post injection and brains were sectioned and immunostained for myelin oligodendrocyte glycoprotein peptide (MOG) , Adenomatous polyposis coli (APC-a maker of mature oligodendrocytes) and *Olig2.* Quantification of APC-positive cells indicated a 2-fold increase in remyelination in the corpus callosum after treatment with BZA, as depicted in Fig. 3.

**Example 6. BZA effects on remyelination kinetics.** Toxic, focal demyelinating injury was induced in the corpus callosum of 8-week old adult mice the rate and extent of remyelination was assessed, using previously described methodology. Demyelination was induced by injecting 1 µl of 1% solution of lysolecithin. Essential to this demyelinating model is the timeline for repair, comprising active demyelination [1-3 days post lesion (dpl)], OPC recruitment (3-7 dpl, peaking at 5 dpl), oligodendrocyte differentiation (7-10 dpl) and active remyelination (14-21 dpl). Oligodendrocyte differentiation and remyelination at 10 dpl was analyzed, allowing assessment of BZA effects on the kinetics of myelin repair. BZA was administered via oral gavage to adult mice at a concentration of 10 mg/kg/day for 7 days following lysolecithin injections. Animals were sacrificed and perfused at 10 dpl. MOG and CC1/APC immunostaining demonstrated enhanced differentiation and significantly more oligodendrocytes in the lesion of BZA-treated mice at 10 dpl when compared to littermate vehicle-treated controls. These findings further demonstrate that BZA greatly promotes differentiation and accelerates the kinetics of remyelination after a demyelinating insult.

**Example 7. Remyelination effects are independent of estrogen receptor activity.**

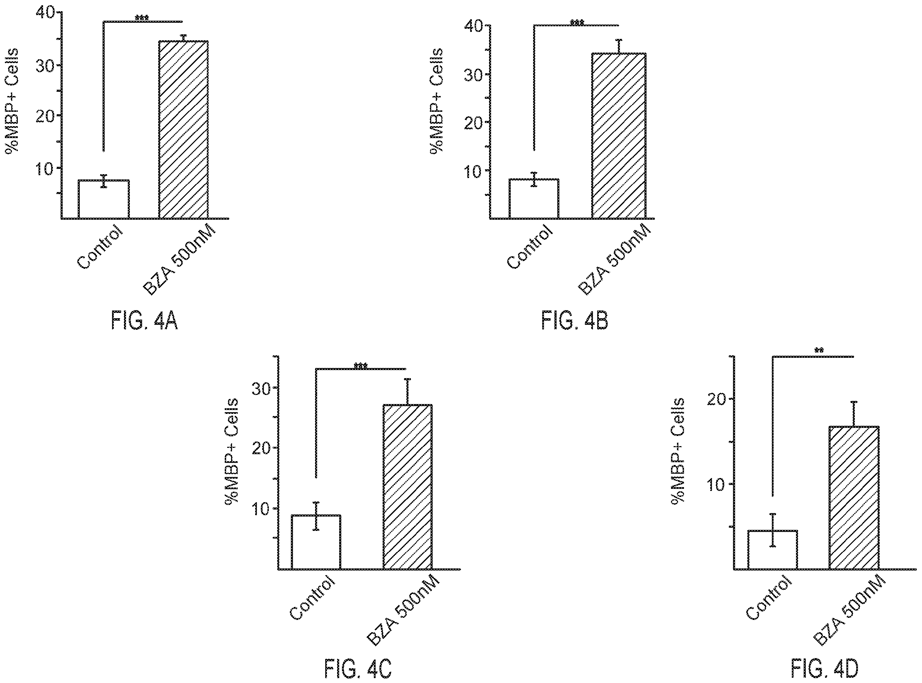

OPCs were isolated from estrogen receptor alpha (ERα) or beta (EHβ) null P7 mice, as well as from double knockout animals. Knockout animals were confirmed via genotyping for the inserted cassette into either the *Esr1* or *Esr2* gene (genes that encode for ERa and ERβ, respectively). OPCs were cultured in isolation, and treated with 500nM BZA for 48 hours. Cells were then permeabilized and stained for MBP (oligodendrocytes), PGFRα (OPCs), and DAPI (cell bodies) MBP/DAPI+ cells/total cells were quantified under 20x magnification. Significance was determined used two-tailed Student's t-test (*p<0.05; **p<0.01; ***p<0.001). BZA significantly enhanced OPC differentiation in wild type (Fig. 4A), ERa null (Fig. 4B) , ERβ null (Fig. 4C), and ERα/ERβ null mice (Fig. 4D), when compared with control. These results demonstrate that ERs are not necessary for SERM remyelinating agents to elicit remyelination effects.

**Example 8. Remyelinating SERMs activate GPR56.** As previous findings demonstrated that SERMs do not act through the estrogen receptors, a bioinformatics screen was conducted to identify the molecular target of the remyelinating SERMs which promotes remyelination, employing a bioinformatics approach which integrates chemical and molecular profiling. Specifically, the analysis was designed to identify non-overlapping targets between estrogen derivatives (which alone have no effects on myelination) and the remyelinating SERMs. The top candidate identified was GPR56, an adhesion GPCR which has previously been implicated as playing a role in oligodendrocyte development. Preliminary data suggests that GPR56 is the molecular target of the remyelinating SERMs for promotion of remyelinating effects.

## Claims

1. A pharmaceutical composition comprising a remyelinating SERM for use in the treatment of a demyelinating condition in a subject, wherein the remyelinating SERM is selected from the group consisting of bazedoxifene, and clomifene.

2. The pharmaceutical composition for use according to Claim 1 , wherein the remyelinating SERM is bazedoxifene.

3. The pharmaceutical composition for use according to Claim 1, further comprising an estrogenic agent.

4. The pharmaceutical composition for use according to Claim 3, wherein
the estrogenic agent is selected from the group consisting of estrogen, estradiol, conjugated estrogens, estriol, estrogen mimic, estrone, ethynil estrogen, and estrogen agonist.

5. The pharmaceutical composition for use according to Claim 1, wherein the demyelinating condition is a condition of the central nervous system.

6. The pharmaceutical composition for use according to Claim 5, wherein
the demyelinating condition of the central nervous system is selected from the group consisting of a myelinoclastic disorder, multiple sclerosis (MS), Devic's disease, an inflammatory demyelinating disease, an acute disseminated encephalomyelitis, a leukodystrophic disorder, central nervous system neuropathy, central pontine myelinolysis, and progressive multifocal leukoencephalopathy.

7. The pharmaceutical composition for use according to Claim 6, wherein the demyelinating condition is MS.

8. The pharmaceutical composition for use according to Claim 1, wherein the demyelinating condition is a condition of the peripheral nervous system.

9. A pharmaceutical composition comprising a remyelinating SERM for use in promoting oligodendrocyte precursor cells in a subject to differentiate into myelin-producing oligodendrocytes, wherein the remyelinating SERM is selected from the group consisting of bazedoxifene, and clomifene.

10. The pharmaceutical composition for use according to Claim 9, wherein
the remyelinating SERM is bazedoxifene.

11. The pharmaceutical composition for use according to Claim 9, further comprising
an estrogenic agent.

12. The pharmaceutical composition for use according to Claim 11, wherein
the estrogenic agent is selected from the group consisting of estrogen, estradiol, conjugated estrogens, estriol, estrogen mimic, estrone, ethynil estrogen, and estrogen agonist.

13. The pharmaceutical composition for use according to Claim 9, wherein
the demyelinating condition is selected from the group consisting of a myelinoclastic disorder, MS, Devic's disease, an inflammatory demyelinating disease, an acute disseminated encephalomyelitis, a leukodystrophic disorder, central nervous system neuropathy, central pontine myelinolysis, progressive multifocal leukoencephalopathy, and a condition of the peripheral nervous system.

## Patentansprüche

1. Pharmazeutische Zusammensetzung, umfassend einen remyelinisierenden SERM, zur Verwendung bei der Behandlung eines demyelinisierenden Zustands in einem Individuum, wobei der remyelinisierende SERM aus der aus Bazedoxifen und Clomifen bestehenden Gruppe ausgewählt ist.

2. Pharmazeutische Zusammensetzung zur Verwendung nach Anspruch 1, wobei der remyelinisierende SERM Bazedoxifen ist.

3. Pharmazeutische Zusammensetzung zur Verwendung nach Anspruch 1, die ferner ein östrogenes Mittel umfasst.

4. Pharmazeutische Zusammensetzung zur Verwendung nach Anspruch 3, wobei das östrogene Mittel aus der aus Östrogen, Östradiol, konjugierten Östrogenen, Östriol, Östrogenmimetikum, Östron, Ethinilöstrogen und Östrogenagonist bestehenden Gruppe ausgewählt ist.

5. Pharmazeutische Zusammensetzung zur Verwendung nach Anspruch 1, wobei der demyelinisierende Zustand ein Zustand des zentralen Nervensystems ist.

6. Pharmazeutische Zusammensetzung zur Verwendung nach Anspruch 5, wobei der demyelinisierende Zustand des zentralen Nervensystems aus der aus myelinoklastischer Störung, Multipler Sklerose (MS), Devic-Krankheit, einer entzündlichen demyelinisierenden Krankheit, einer akuten disseminierten Enzephalomyelitis, einer Leukodystrophie-Störung, Neuropathie des zentralen Nervensystems, zentraler pontiner Myelinolyse und progressiver multifokaler Leukoenzephalopathie bestehenden Gruppe ausgewählt ist.

7. Pharmazeutische Zusammensetzung zur Verwendung nach Anspruch 6, wobei der demyelinisierende Zustand MS ist.

8. Pharmazeutische Zusammensetzung zur Verwendung nach Anspruch 1, wobei der demyelinisierende Zustand ein Zustand des peripheren Nervensystems ist.

9. Pharmazeutische Zusammensetzung, umfassend einen remyelinisierenden SERM, zur Verwendung bei der Förderung der Differenzierung von Oligodendrozyten-Vorläuferzellen in einem Individuum in Myelin-produzierende Oligodendrozyten, wobei der remyelinisierende SERM aus der aus Bazedoxifen und Clomifen bestehenden Gruppe ausgewählt ist.

10. Pharmazeutische Zusammensetzung zur Verwendung nach Anspruch 9, wobei der remyelinisierende SERM Bazedoxifen ist.

11. Pharmazeutische Zusammensetzung zur Verwendung nach Anspruch 9, die ferner ein östrogenes Mittel umfasst.

12. Pharmazeutische Zusammensetzung zur Verwendung nach Anspruch 11, wobei das östrogene Mittel aus der aus Östrogen, Östradiol, konjugierten Östrogenen, Östriol, Östrogenmimetikum, Östron, Ethinilöstrogen und Östrogenagonist bestehenden Gruppe ausgewählt ist.

13. Pharmazeutische Zusammensetzung zur Verwendung nach Anspruch 9, wobei der demyelinisierende Zustand aus der aus einer myelinoklastischen Störung, MS, Devic-Krankheit, einer entzündlichen demyelinisierenden Krankheit, einer akuten disseminierten Enzephalomyelitis, einer Leukodystrophie-Störung, Neuropathie des zentralen Nervensystems, zentraler pontiner Myelinolyse, progressiver multifokaler Leukoenzephalopathie und einem Zustand des peripheren Nervensystems bestehenden Gruppe ausgewählt ist.

## Revendications

1. Composition pharmaceutique comprenant un MSRE de remyélinisation, pour une utilisation dans le traitement d'une condition démyélinisante chez un sujet, dans laquelle le MSRE de remyélinisation est choisi dans le groupe constitué par le bazédoxifène et le clomifène.

2. Composition pharmaceutique pour une utilisation selon la revendication 1, dans laquelle le MSRE de remyélinisation est le bazédoxifène.

3. Composition pharmaceutique pour une utilisation selon la revendication 1, comprenant en outre un agent œstrogénique.

4. Composition pharmaceutique pour une utilisation selon la revendication 3, dans laquelle l'agent œstrogénique est choisi dans le groupe constitué par un œstrogène, l'estradiol, les œstrogènes conjugués, l'estriol, un mimétique d'œstrogène, l'estrone, un éthinyl-œstrogène, et un agoniste d'œstrogène.

5. Composition pharmaceutique pour une utilisation selon la revendication 1, dans laquelle la condition démyélinisante est une condition du système nerveux central.

6. Composition pharmaceutique pour une utilisation selon la revendication 5, dans laquelle la condition démyélinisante du système nerveux central est choisie dans le groupe constitué par un trouble myélinoclastique, la sclérose en plaques (SEP), la maladie de Devic, une maladie démyélinisante inflammatoire, l'encéphalomyélite aiguë disséminée, un trouble leucodystrophique, une neuropathie du système nerveux central, la myélinolyse centropontine, et la leucoencéphalopathie multifocale progressive.

7. Composition pharmaceutique pour une utilisation selon la revendication 6, dans laquelle la condition démyélinisante est la SEP.

8. Composition pharmaceutique pour une utilisation selon la revendication 1, dans laquelle la condition démyélinisante est une condition du système nerveux périphérique.

9. Composition pharmaceutique comprenant un MSRE de remyélinisation, pour une utilisation afin de favoriser la différentiation de cellules précurseurs des oligodendrocytes chez un sujet en oligodendrocytes producteurs de myéline, dans laquelle le MSRE de remyélinisation est choisi dans le groupe constitué par le bazédoxifène et le clomifène.

10. Composition pharmaceutique pour une utilisation selon la revendication 9, dans laquelle le MSRE de remyélinisation est le bazédoxifène.

11. Composition pharmaceutique pour une utilisation selon la revendication 9, comprenant en outre un agent œstrogénique.

12. Composition pharmaceutique pour une utilisation selon la revendication 11, dans laquelle l'agent œstrogénique est choisi dans le groupe constitué par un œstrogène, l'estradiol, les œstrogènes conjugués, l'estriol, un mimétique d'œstrogène, l'estrone, un éthinyl-œstrogène, et un agoniste d'œstrogène.

13. Composition pharmaceutique pour une utilisation selon la revendication 9, dans laquelle la condition démyélinisante est choisie dans le groupe constitué par un trouble myélinoclastique, la SEP, la maladie de Devic, une maladie démyélinisante inflammatoire, l'encéphalomyélite aiguë disséminée, un trouble leucodystrophique, une neuropathie du système nerveux central, la myélinolyse centropontine, la leucoencéphalopathie multifocale progressive, et une condition du système nerveux périphérique.
